# EUROPEAN PATENT SPECIFICATION

(11) **EP 4 241 656 B1**
(45) Date of publication and mention of the grant of the patent: **28.10.2026**
(21) Application number: 21906777.4
(22) Date of filing: 28.07.2021
(51) Int. Cl.: A61B 5/00, A61B 5/145, A61B 5/155, A61B 5/15, A61B 5/1495

(54) **BODY-ATTACHABLE DEVICE FOR CONTINUOUSLY MEASURING BIOMETRIC DATA**
AM KÖRPER ANBRINGBARE VORRICHTUNG ZUR KONTINUIERLICHEN MESSUNG BIOMETRISCHER DATEN
DISPOSITIF POUVANT ÊTRE FIXÉ AU CORPS POUR MESURER EN CONTINU DES DONNÉES BIOMÉTRIQUES

(30) Priority: 14.12.2020 KR 20200174277
(43) Date of publication of application: 13.09.2023
(73) Proprietor: i-Sens, Inc., Seoul 06646 (KR)
(72) Inventor: KANG, Young Jea, Seoul 06646 (KR); PARK, Jeong Je, Seoul 06646 (KR); LEE, Seung Chun, Seoul 06646 (KR)
(74) Representative: BCKIP Part mbB
(86) International application number: PCT/KR2021/009795
(87) International publication number: WO 2022/131472

(56) References cited:
- EP-A1- 3 733 052
- EP-B1- 2 256 495
- WO-A1-2014/145001
- JP-A- 2019 530 492
- KR-A- 20050 110 711
- KR-A- 20110 109 603
- KR-A- 20180 074 559
- KR-A- 20200 016 590

## Description

### Technical Field

The present invention relates to a body-attachable device attached to the body to continuously measure biometric data and, more particularly, to a body-attachable device capable of: accurately providing an alarm to a user by deciding upon an alarm mode on the basis of the state of communication establishment between a receiver and the body-attachable device and providing the alarm to the user; and responding quickly in an emergency to protect the health of the user by providing an alarm in an alarm mode which the user can promptly become aware of in accordance with the state of communication establishment with the receiver or the importance of the alarm.

### Background

Diabetes is a leading cause of death worldwide and is a factor which causes physical impairments, and therefore many people develop health problems due to diabetes. In particular, diabetes is a serious disease which causes heart and kidney disease, blindness, neural damage and high blood pressure. Looking at long-term clinical studies, the occurrence of complications can be significantly reduced by means of adequately controlling blood glucose levels. Therefore, it is important to continuously manage diabetes, and a major factor thereof is self-monitoring of blood glucose levels.

Due to such requirements, personal biometric devices which can directly test the blood glucose of the user are widely supplied to and used by users. Typical blood glucose measuring devices measure a user's blood glucose level by smearing the user's blood onto a sensor strip in the form of a test paper. That is, the sensor strip smeared with blood is inserted into the blood glucose measuring device and the blood glucose level measured via the sensor strip is displayed on the blood glucose measuring device.

At this time, the blood glucose measuring device receives an electrical signal generated by an electrochemical reaction of the collected blood and the reactants inside the sensor strip and so measures the blood glucose level. This blood-collecting blood glucose meter (finger prick method) helps with a diabetes patient's blood glucose management, but there is a problem in that it is difficult to accurately understand the frequently changing blood glucose value since only the result at the time of measurement is indicated.

Diabetes patients generally move between hyperglycaemic and hypoglycaemic states, and with emergencies occurring when in the hypoglycaemic state, and when the supply of glucose does not last for a long time, they may lose consciousness or, in the worst case, may lose their lives. Therefore, the prompt detection of a hypoglycaemic state is very important to diabetes patients. But blood-collecting blood glucose meters which intermittently measure blood glucose have obvious limitations.

In order to overcome the limitations of such blood-collecting blood glucose meters, a continuous blood glucose measuring system (Continuous Glucose Monitoring System, CGMS) which is inserted into the body to measure blood glucose at intervals of a few minutes has been developed, and this can be used to manage diabetic patients and easily cope with emergency situations.

The continuous blood glucose measuring system comprises: a body-attachable unit which is inserted into the body and is for collecting test material such as a bodily fluid of the user and measuring the blood glucose; and a receiver for communicating with the body-attachable device and displaying the blood glucose data measured by the body-attachable unit.

The receiver not only outputs the blood glucose data received from the body-attachable device to the user, but also can provide an alarm to the user when the user has hyperglycaemia or hypoglycaemia on the basis of the blood glucose data, or can monitor the service condition of the body-attachable device and provide an alarm to the user.

For example, the receiver can give an alarm for replacing the body-attachable device by monitoring the service life of the body-attachable device, or an alarm for requesting calibration of the body-attachable unit, or an alarm for notification of danger when the user's blood glucose is hyperglycaemic or hypoglycaemic.

To be more specific, body-attachable devices cannot be permanently used and have a fixed term of life, for example, a service life of 15 days or 1 month, so replacement is necessary when the service life expires. If the service life has ended, the body-attachable device does not measure the blood glucose in the body after this endpoint and there are also no more data transmissions to the receiver. There are also cases where the power source of the body-attachable device is automatically turned off depending on the circumstances.

Therefore, since users cannot receive any more blood glucose data from the body-attachable device after the service life of the body-attachable device has ended, the user should be notified to replace the body-attachable device in advance before the service life of the body-attachable device ends.

Meanwhile, in order to provide accurate blood glucose data to the user, the blood glucose data received from the body-attachable device must be initially calibrated using a separate blood glucose measuring device, and thereafter must continue to be calibrated, at fixed periods during the service life of the body-attachable device, with a reference blood glucose value measured by a separate blood glucose measuring device. If there is no calibration of the blood glucose value measured by the body-attachable device, the reliability of the blood glucose value measured by the body-attachable device falls, and therefore an alarm for periodically calibrating the blood glucose value should be provided to the user.

Meanwhile, the user should be alerted of the danger of hyperglycaemia or hypoglycaemia if the blood glucose value measured by the body-attachable device is compared to a target blood glucose value set by the user and the user's blood glucose value exceeds the target blood glucose value, or if the user's blood glucose value exceeds a set blood glucose value upper limit or blood glucose value lower limit.

However, if the user is not carrying a receiver the user cannot be made aware of the alarm provided via the receiver, or if the user is asleep the user cannot be made aware of the alarm provided via the receiver. In particular, hypoglycaemia can occasionally occur during sleep, so even if a hypoglycaemia alarm is provided via the receiver, the user may be at great risk because the user fails to become aware of the alarm noise while asleep or because the receiver and user are far apart from each other.

Meanwhile, the receiver can output various alarms to the user, but the user will feel fatigue due to the alarms or avoid or ignore responding to the alarms as a result of various alarms being provided to the user or repetitive additional alarms being provided to the user, and so, in the case of an important alarm, it is necessary to provide the alarm in such a manner that the user can immediately be aware of the alarm.

In addition, set alarms are output from the receiver even when the user is reluctant to inform the people around them of their use of the body-attachable device or does not want to be disturbed by the alarm during a meeting, so there is the problem of alarms being provided to the user against the user's wishes.

WO 2014/145001 A1 discloses a device to monitor an analyte in body fluid including an on body electronic device with an analyte sensor and a display device for displaying information to the user. When a programmed alarm condition is detected, such as glucose levels falling outside a specific range, an alarm is triggered. The user can view this alert via the user interface

on the on body device. If the display device is connected, the alert is also sent there, allowing the user to take corrective action. Another device for providing different alarms based on measured physiological data is known from EP 2 256 495 B1.

### Details of the Invention

### Problem to be Solved

The present invention aims to resolve the problems of the conventional method for providing an alarm pertaining to continuous biometric data as mentioned above, and an object of the present invention is to provide a body-attachable device which is capable of deciding upon an alarm mode on the basis of the state of communication establishment between a receiver and the body-attachable device and accurately providing an alarm to a user in accordance with the alarm mode decided upon.

Another object of the present invention is to provide a body-attachable device which is capable of deciding upon an alarm mode by determining the importance of the alarm when the measured biometric data or the service condition of the body-attachable device satisfies an alarm condition and accurately and promptly providing an alarm to the user in the alarm mode decided upon.

Yet another object of the present invention is to provide a body-attachable device which allows the user to respond quickly to protect their health by providing an alarm in an alarm mode which the user can promptly become aware of in accordance with the state of communication establishment with the receiver or the importance of the alarm.

### Solution to the Problem

In order to achieve the objects of the present invention, a body-attachable device according to claim 1 is provided.

The alarm unit according to the present invention is characterized in that the alarm unit determines the state of communication establishment between the body-attachable device and the receiver, and decides upon the alarm mode in accordance with the state of communication establishment and provides an alarm to the user in the alarm mode decided upon.

Here, the alarm unit is characterized in that the alarm unit determines whether the body-attachable device and the receiver are in communication with each other on the basis of whether a data request message is received from the receiver in response to a pop-up message periodically transmitted via the communication unit.

The alarm unit according to the present invention is characterized in that the alarm unit determines the importance of the alarm in accordance with the service condition of the body-attachable device or the measured biometric data which satisfies the defined alarm condition, and decides the alarm mode on the basis of the determined alarm importance and state of communication establishment and provides an alarm to the user in the alarm mode decided upon.

Here, the alarm mode is characterized in that the alarm mode is any one of: a receiver mode in which an alarm is output to the user from a receiver; an autonomous mode in which an alarm is output to the user from the body-attachable device; and a simultaneous mode in which alarms are output to the user simultaneously from the receiver and the body-attachable device.

Preferably, the alarm unit according to the present invention is characterized in that the alarm unit further comprises an output unit, and the alarm unit generates different alarm signals in accordance with the importance of the alarm and outputs same through the output unit.

Here, the alarm condition is characterized in that the alarm condition is any one of: a calibration alarm for requesting input of a reference blood glucose value; a replacement alarm condition for requesting replacement of the body-attachable device; or a danger alarm for notification about a dangerous state on the basis of the biometric data.

One embodiment of the present invention is characterized in that, in the case of the danger alarm, the alarm condition is set by input from the user.

Another embodiment of the present invention is characterized in that, in the case of the danger alarm, the alarm condition is set from previously measured biometric data.

Here, the alarm unit is characterized in that, on the basis of the state of communication establishment with the receiver, the alarm unit performs control to output an alarm in the receiver mode when communication is established with the receiver, and performs control to output an alarm in the autonomous mode when the communication with the receiver is interrupted.

Here, the alarm unit is characterized in that, the alarm unit performs control to output alarms in the simultaneous mode when communication is established with the receiver and the importance of the alarm is determined to be high.

### Advantages of the Invention

The advantages of the body-attachable device according to the present invention which is disposed on a user's body to measure the user's biometric data and transmit the measured biometric data to a receiver are as follows.

Firstly, the body-attachable device according to the present invention is capable of accurately providing an alarm to a user by deciding upon an alarm mode on the basis of the state of communication establishment between a receiver and the body-attachable device and providing an alarm to the user.

Secondly, the body-attachable device according to the present invention is capable of accurately and promptly providing an alarm to the user by deciding upon an alarm mode by determining the importance of the alarm when the measured biometric data or service condition of the body-attachable device satisfies an alarm condition.

Thirdly, the body-attachable device according to the present invention is capable of protecting the health of the user or accurately measuring biometric data in an emergency by providing an alarm in an alarm mode which the user can promptly become aware of in accordance with the state of communication establishment with the receiver or the importance of the alarm.

### Brief Description of the Drawings

FIG. 1 is a drawing for describing a continuous biometric data measuring system according to the present invention.
FIG. 2 depicts an example of the body-attachable device according to the present invention.
FIG. 3 is a drawing for describing an example of a calibration alarm condition.
FIG. 4 is a drawing for describing an example of a replacement alarm condition.
FIG. 5 is a drawing for describing an example of a danger alarm condition.
FIG. 6 is a function block diagram for describing the body-attachable device according to the present invention.
FIG. 7 describes an example of establishing communication with a receiver via Bluetooth.
FIG. 8 is a function block diagram for describing an example of the alarm unit according to the present invention.
FIG. 9 is a drawing for describing an example of outputting an alarm to a user in autonomous mode or simultaneous mode.

### Specific Details for Implementing the Invention

It should be noted that the technical terms used in the present invention are merely used to describe a specific embodiment of the invention, and are not intended to limit the present invention. Furthermore, the technical terms used in the present invention should be interpreted as having the meaning commonly understood by a person skilled in the art to which the present invention belongs unless specifically defined otherwise in the present invention, and should not be interpreted as having an excessively inclusive meaning or an excessively limited meaning. Furthermore, when a technical term used in the present invention is an erroneous technical term that fails to accurately express a concept of the present invention, it should be understood as having been replaced by a technical term that can be correctly understood by those skilled in the art.

Furthermore, expressions in the singular used in the present invention include expressions in the plural unless clearly intended otherwise in context. In the present invention, terms such as "consisting of" or "comprising" should not necessarily be interpreted as including all the constituent elements or all the steps described in the invention, and some constituent elements or some steps may not be included, or additional constituent elements or steps may be further included.

Furthermore, it should be noted that the accompanying drawings are merely to facilitate the understanding of the concept of the present invention, and the concept of the present invention should not be construed as being limited by the accompanying drawings.

Hereinbelow, the body-attachable device according to the present invention shall be described in greater detail with reference to the accompanying drawings.

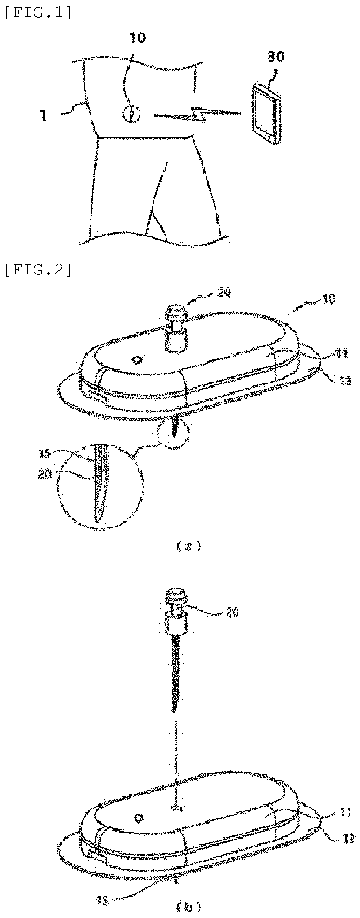

FIG. 1 is a drawing for describing a continuous biometric data measuring system according to the present invention.

Referring to FIG. 1, the continuous biometric data measuring system according to one embodiment of the present invention comprises a body-attachable device (10) and a receiver (30).

The body-attachable device (10) is attached to the body (1), and, when the body-attachable device (10) is attached to the body, one end of a sensor of the body-attachable device (10) is inserted into the skin to periodically extract a bodily fluid in the body to continuously measure biometric data such as blood glucose. Here, the body-attachable device (10) comprises a sensor which is inserted into the skin to extract a bodily fluid, and a transmitter which transmits the biometric data to the receiver (30) after the biometric data has been measured from the bodily fluid. The sensor and transmitter are assembled together at the factory to manufacture the body-attachable device, and the body-attachable device (10) is further mounted on an applicator (not depicted) which attaches the body-attachable device (10) to the body.

The user removes the applicator from its wrapping and then positions the applicator on the area of the body where the body-attachable device is to be mounted, and then places the body-attachable device on the skin using the applicator.

After placement of the body-attachable device (10) on the skin is completed, the body-attachable device (10) and the receiver (30) are connected in communication with each other so that the biometric data measured by the body-attachable device (10) is transmitted/received.

Here, the receiver (30) can establish communication with the body-attachable device (10), and a dedicated terminal or smartphone or the like, which can receive biometric data from the body-attachable device (10) and provide the biometric data to a user, can be used. The receiver is not limited to smartphones, tablet PCs or notebooks, and the type of terminal does not matter as long as the terminal includes a communication function and a program or application may be installed.

The receiver (30) periodically receives biometric data from the body-attachable device (10) after establishing communication with the body-attachable device (10), and calibrates the received biometric data and outputs the biometric data to the user.

The receiver (30) provides an alarm to the user on the basis of the service condition of the body-attachable device (10) or provides an alarm to the user on the basis of the biometric data received from the body-attachable device (10).

For example, the receiver (30) provides a danger alarm to the user on the basis of the biometric data received from the body-attachable device (10) when the biometric data exceeds a set value, or provides a replacement alarm to the user when the time for replacing the body-attachable device (10) which has a limited service life arrives, or provides a calibration alarm to the user when the time for inputting calibration information for the biometric data measured via the body-attachable device (10) arrives.

The user can check the alarm via the receiver (30) and can clear the alarm by performing a required action or inputting the required information depending on the kind of alarm.

However, when the user is not carrying the receiver (30) or is far apart from the receiver (30), the user cannot receive the alarm from the body-attachable device (10) and therefore cannot be made aware of important alarms. Furthermore, when an alarm situation occurs while the user is asleep, it is difficult for the user to be aware of the alarm provided via the receiver (30).

The body-attachable device (10) according to the present invention decides the alarm mode taking the state of communication establishment with the receiver (30) into consideration, and is configured such that, depending on the alarm mode decided upon, the user can check the alarm via the receiver (10) or the user can check the alarm output directly from the body-attachable device (10).

Furthermore, the body-attachable device (10) according to the present invention decides the alarm mode in accordance with not only the state of communication establishment but also the importance of the alarm, and, in the case of an important alarm, can be configured to output an alarm directly to the user from the body-attachable device (10) or simultaneously output alarms to the user from the receiver (30) and the body-attachable device so that the user can accurately and promptly become aware of the alarm and respond quickly to an alarm situation.

Various pieces of the user's biometric data can be continuously measured via the body-attachable unit (10), but blood glucose data is described hereinbelow as an example of biometric data.

FIG. 2 depicts an example of the body-attachable device according to the present invention.

Referring to FIG. 2, the body-attachable device is configured such that the body-attachable device (10) provided with a sensor member (15), which is inserted into the body to continuously measure the blood glucose, is attached to the body via an applicator (not depicted).

In the applicator, the sensor member (15) is disposed in a hollow insertion needle (20) that is open on one side, and, when the insertion needle (20) is inserted into the body, it is inserted into the body together with the sensor member (15), and, when the insertion needle (20) is removed from the body, only the sensor member (15) is left inserted into the body.

The body-attachable device (10) is provided with an adhesive member (13) on the bottom of a housing (11), whereby the body-attachable device (10) is stably fixed and attached to the body with the sensor member (15) inserted into the body. The body-attachable device (10) is configured to be inserted and attached to the body by operating the applicator and to continue to periodically measure the blood glucose from the body, and the blood glucose measurement data periodically measured via the body-attachable device (10) is transmitted to a separate receiver (30) and output.

The body-attachable device (10) is formed to be attachable to the body so as to enable the extraction of bodily fluid and periodic measurement of the blood glucose, and is formed to be able to transmit the blood glucose measurement results to the external receiver (30). In such a body-attachable device (10), the sensor member (15) of which one end is inserted into the body, and the transmitter (not depicted) which can wirelessly communicate with the receiver (30), can be disposed inside the housing (11).

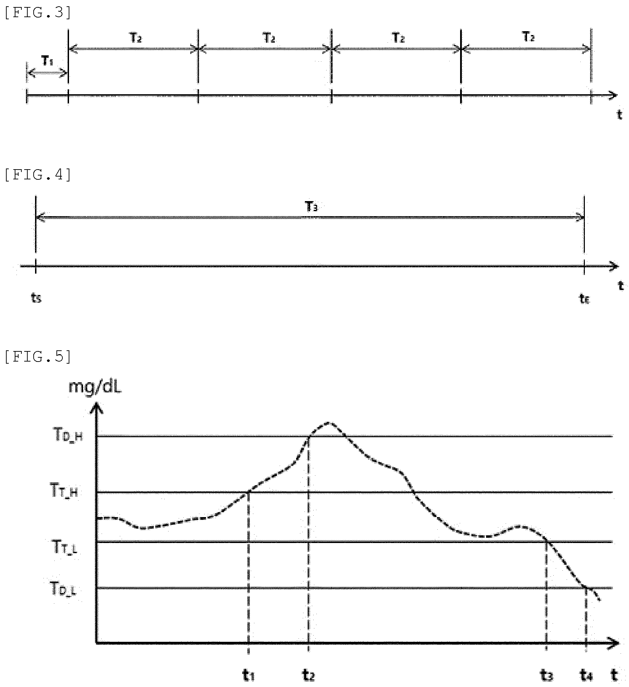

FIGS. 3 to 5 are drawings for describing examples of alarm conditions.

With respect to the calibration alarm condition referred to in FIG. 3, the body-attachable device goes through a stabilization period (T₁) after being inserted into the body, and then extracts bodily fluid from the body and continuously measures blood glucose data during a fixed service life (e.g., 15 days, 1 month, etc.). In order for the body-attachable device to accurately measure blood glucose data, the periodically measured blood glucose data should be calibrated with blood glucose data measured using a separate blood glucose measuring device and test strip as a reference blood glucose value, and if it is not calibrated with a reference blood glucose value every calibration period, accurate blood glucose values cannot be provided to the user.

Therefore, when a calibration period (T₂) arrives, an alarm can be given so that the user inputs data on a reference blood glucose value in advance so as to satisfy the calibration alarm condition.

With respect to the replacement alarm condition referred to in FIG. 4, since the body-attachable device cannot be permanently used and has a fixed service life (T₃), replacement is necessary when the service life expires. If the service life has ended, the body-attachable unit does not measure the blood glucose in the body after this endpoint and there are also no more data transmissions to the receiver.

Therefore, when the time for replacing the body-attachable device arrives because the user cannot receive further blood glucose data from the body-attachable device after the service life of the body-attachable device has ended, an alarm can be given so that the user replaces the body-attachable device before the end of the service life so as to satisfy the replacement alarm condition.

Meanwhile, with respect to the danger alarm condition referred to in FIG. 5, a target blood glucose range can be set by setting a target high blood glucose value (T_{T_H}) and a target low blood glucose value (T_{T_L}) for the user in accordance with the condition of the user's body. When the blood glucose value measured in real-time by the body-attachable device is outside of the target blood glucose range, the hyperglycaemia or hypoglycaemia alarm condition is satisfied which enables the user to respond to the hyperglycaemia or hypoglycaemia.

The danger alarm conditions depending on the alarm mode can be set to be different in accordance with the field that the present invention is applied to. For example, there may be a setting in which the danger alarm condition is satisfied when outside of the target blood glucose range in an alarm mode for outputting an alarm to the user from the receiver, and there may be a setting in which the danger alarm condition is satisfied when the user's blood glucose value exceeds the dangerous hyperglycaemia value (T_{D_H}) or is equal to or less than the dangerous hypoglycaemia value (T_{D_L}) in an alarm mode for outputting an alarm to the user directly from the body-attachable device or in an alarm mode for outputting alarms to the user from the body-attachable device and the receiver simultaneously.

In another example, there may be a setting in which the immediate danger alarm condition is satisfied when the user's blood glucose value exceeds the dangerous hyperglycaemia value or is equal to or less than the dangerous hypoglycaemia value in an alarm mode for outputting an alarm to the user from the receiver, and there may be a setting in which the danger alarm condition is satisfied when the user's blood glucose value exceeds the dangerous hyperglycaemia value or is equal to or less than the dangerous hypoglycaemia value during a fixed period or for more than a fixed number of times in an alarm mode for outputting an alarm to the user directly from the body-attachable device or in an alarm mode for outputting alarms to the user from the body-attachable device and the receiver simultaneously.

That is, the alarm condition in an alarm mode for outputting an alarm to the user from the receiver and the alarm condition in an alarm mode for outputting an alarm to the user directly from the body-attachable device are set to be different, such that an alarm is provided directly to the user via the body-attachable device only in an emergency situation so that the user fatigue due to repetitive alarms is reduced and at the same time the user can accurately become aware of and quickly respond to an emergency situation even if the receiver is far away or during sleep.

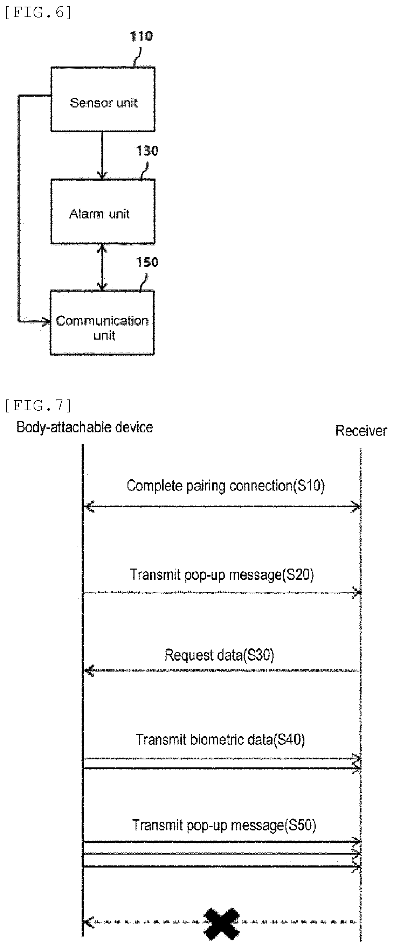

FIG. 6 is a function block diagram for describing an example of a body-attachable device according to the present invention.

To describe the body-attachable device according to the present invention in more detail with reference to FIG. 6, the device comprises: a sensor unit (110) which extracts bodily fluid from the body to generate blood glucose data; a communication unit (150) which establishes communication with the receiver and transmits the generated blood glucose data to the receiver; and an alarm unit (130) which determines whether a set alarm condition is satisfied on the basis of the measured blood glucose data or the service condition of the body-attachable device, and when the set alarm condition is satisfied, provides an alarm to the user.

In more detail, the communication unit (150) is in communication with the receiver and transmits/receives data to/from the receiver when the body-attachable device is disposed on the body. The communication unit (150) can be in communication with the receiver via wired or wireless communication depending on the field in which the present invention is applied, for example, be in communication with the receiver via USB communication, infra-red communication or Bluetooth communication.

FIG. 7 describes an example of establishing communication with a receiver via Bluetooth, and data such as identification data of the body-attachable device is transmitted/received to /from the receiver and pairing is performed to connect and initial communication is established (S10). Thereafter, the communication unit (150) can transmit blood glucose data measured by the sensor unit (110) to the receiver, in real-time, either periodically or when there is a request from the receiver. The communication unit (150) first transmits a pop-up message to the receiver (S20) before transmitting blood glucose data to the receiver, and when a data request message is received from the pop-up message in response to the pop-up message (S30), transmits the measured blood glucose data to the receiver (S40).

Next, when the transmission period of the blood glucose data arrives, the communication unit (150) again transmits the pop-up message to the receiver (S50), and, if a data request message cannot be received from the receiver in response to the pop-up message, stores the measured blood glucose data and then, in the next transmission period, transmits the pop-up message to the receiver again and transmits blood glucose data including the untransmitted blood glucose data to the receiver.

Referring to FIG. 6 again, the alarm unit (130) determines the service condition of the body-attachable device. For example, the alarm unit determines that the body-attachable device is in use from the time point at which initial communication is established with the receiver, and counts down the service life or counts down the calibration period of the body-attachable device. Furthermore, the alarm unit (130) determines the state of communication establishment with the receiver on the basis of whether the data request message is received from the receiver in response to the pop-up message.

The alarm unit (130) determines whether the alarm condition is satisfied on the basis of the service condition of the body-attachable device or on the basis of the measured blood glucose data, and, when the alarm condition is satisfied, decides the alarm mode on the basis of the state of communication establishment with the receiver. Preferably, the alarm unit (130) can determine the importance of the alarm on the basis of the service condition or blood glucose which satisfies the alarm condition, and can decide the alarm mode on the basis of the importance of the alarm as well as the state of communication establishment.

Depending on the alarm mode decided upon, the alarm unit (130) performs control to output an alarm to the user via the receiver, or performs control to output an alarm to the user directly from the body-attachable device, or performs control to output alarms to the user simultaneously from the receiver and the body-attachable device.

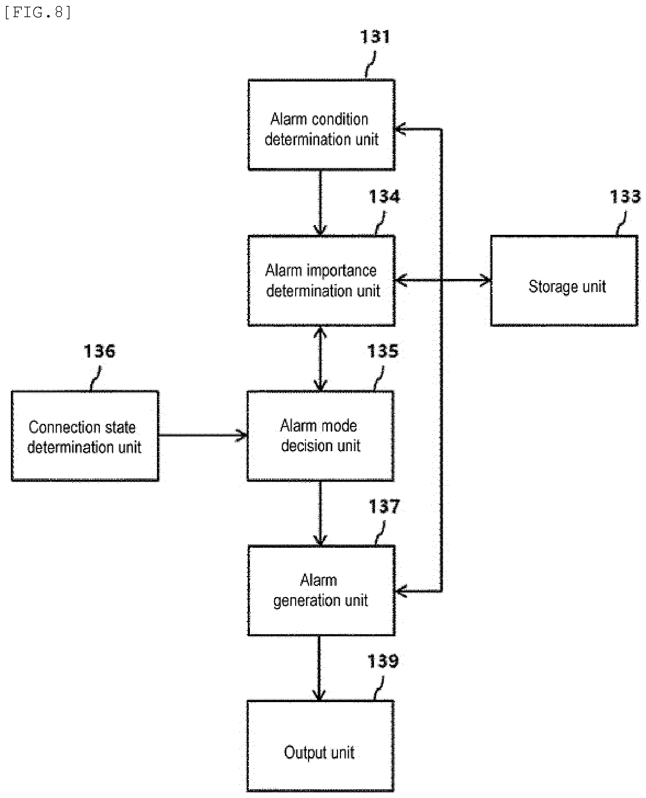

FIG. 8 is a function block diagram for describing an example of the alarm unit according to the present invention.

To describe this in more detail with reference to FIG. 8, an alarm condition determination unit (131) determines whether an alarm condition, in which the service condition of the body-attachable device is preset in a storage unit (133), is satisfied on the basis of the service condition of the body-attachable device or whether an alarm condition, in which the measured blood glucose data is preset in the storage unit (133), is satisfied. Preferably, the alarm condition can be set to receive a value set by the user through the receiver.

For example, the data relating to the service life and the initial use start point of the body-attachable device is stored in the storage unit (133), and the alarm condition determination unit (131) can determine that the replacement alarm condition is satisfied when the replacement time of the body-attachable device arrives on the basis of the service life and the initial use start point.

In another example, a hyperglycaemia threshold value and hypoglycaemia threshold value are stored in the storage unit (133), and the alarm condition determination unit (131) can determine that the danger alarm condition is satisfied when the user's blood glucose value exceeds the hyperglycaemia threshold value or equal to or less than the hypoglycaemia threshold value on the basis of the measured blood glucose data.

In another example, the data relating to the initial use start point and calibration period of the body-attachable device is stored in the storage unit (133), and the alarm condition determination unit (131) can determine that the calibration alarm condition is satisfied when the calibration period arrives on the basis of the initial use start point and the calibration period.

An alarm importance determination unit (134) determines the importance of the alarm condition preset in the storage unit (133) when the alarm condition is satisfied. For example, the replacement alarm condition for notifying that the replacement condition has arrived can be set as having an alarm importance of "normal", and the replacement alarm condition for notifying that the service life has elapsed can be set as having an alarm importance of "high". In yet another example, when the user's blood glucose value exceeds a first hyperglycaemia threshold value, it can be set as having an alarm importance of "normal", and when the user's blood glucose value exceeds a second hyperglycaemia threshold value, it can be set as having an alarm importance of "high" (here, the second hyperglycaemia threshold value is set to be higher than the first hyperglycaemia threshold value).

When an alarm condition is satisfied, an alarm mode decision unit (135) decides the alarm mode on the basis of the state of communication establishment between the body-attachable device and the receiver as determined by a connection state determination unit (136). Preferably, the alarm mode decision unit (135) can decide the alarm mode on the basis of the alarm importance of the alarm condition as well as the state of communication establishment.

Here, the alarm mode can be: a receiver mode in which an alarm is output to the user from the receiver; an autonomous mode in which an alarm is output to the user from the body-attachable device; and a simultaneous mode in which alarms are output to the user simultaneously from the receiver and the body-attachable device, and the alarm mode decision unit (135) decides upon any one of the receiver mode, autonomous mode and simultaneous mode as the alarm mode on the basis of the alarm importance of the alarm condition and the state of communication establishment.

For example, when in a state where communication with the receiver is established, the alarm mode is decided to be the receiver mode and, when in a state where communication with the receiver is interrupted, the alarm mode is decided to be the autonomous mode.

In yet another example, when the alarm importance is "high", the alarm mode is decided to be the simultaneous mode even in a state where communication with the receiver is established.

An alarm generation unit (137) generates an alarm signal in the alarm mode decided upon when the alarm condition is satisfied. Alarm types according to the alarm condition or alarm importance are stored in the storage unit (133), and the alarm generation unit (137) searches for the alarm type according to the alarm condition or alarm importance stored in the storage unit (133) and generates an alarm signal using the found alarm type.

An output unit (139) outputs the alarm signal generated by the alarm generation unit (137) when the alarm mode is the autonomous mode or the simultaneous mode. Here, the output unit

(139) may use, for example, a speaker for output of audio, a vibrator for output of vibrations, or a light-emitting unit for emitting light or may use a combination thereof.

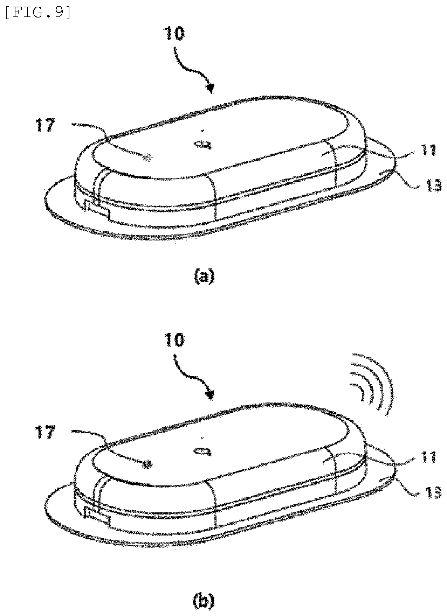

FIG. 9 is a drawing for describing an example of outputting an alarm to a user in autonomous mode or simultaneous mode.

When it is decided that the alarm mode is the autonomous mode or simultaneous mode, an alarm signal is generated as the alarm type set in accordance with the alarm condition and alarm importance and an alarm is directly output to the user from the body-attachable device.

As depicted in FIG. 9(a), when the alarm type set in accordance with the alarm condition (calibration period arrival) and alarm importance (normal) in the autonomous mode is a light, a green light is output via the output unit (17).

As depicted in FIG. 9(b), when the alarm type set in accordance with the alarm condition (hyperglycaemia) and alarm importance (high) in the simultaneous mode is a combination (light, vibration, noise), a red light, a noise and a vibration are output together via the output unit (17).

Meanwhile, the embodiments of the present invention described above may be written as a computer-executable program and may be implemented in a general-purpose digital computer that operates the program using a computer-readable recording medium.

Computer-readable recording media include storage media such as magnetic storage media (e.g., ROM, floppy disk, hard disk, etc.), optical reading media (e.g., CD-ROM, DVD, etc.), and carrier waves (e.g., transmission over the Internet).

The present invention has been described with reference to the embodiments depicted in the drawings, but the embodiments are merely an example, and it should be understood by those skilled in the art that various modifications and other equivalent embodiments are possible. Therefore, the true technical scope of protection for the present invention should be determined by the accompanying claims.

## Claims

1. A body-attachable device (10) configured to be attached to a user's body,
comprising:
a sensor unit (110) configured to be partially insertable into the user's body to measure the user's biometric data;
a communication unit configured to establish communication with a receiver (30) and to transmit the measured biometric data to the receiver (30); and
an alarm unit (130) configured to:
determine a state of communication establishment between the body-attachable device (10) and the receiver (30);
determine whether a set alarm condition is satisfied on the basis of a service condition of the body-attachable device or the measured biometric data,
determine an importance of an alarm in accordance with the service condition of the body-attachable device (10) or the measured biometric data which satisfies the alarm condition,
wherein the alarm condition is any one of: a calibration alarm condition for requesting input of a reference blood glucose value; a replacement alarm condition for requesting replacement of the body-attachable device (10); or a danger alarm condition for notification about a dangerous state on the basis of the biometric data; and **characterized in that** the alarm unit is further configured to:
decide upon an alarm mode on the basis of the determined state of communication establishment and the determined alarm importance, and provide, when the set alarm condition is satisfied, an alarm to the user in the alarm mode decided upon,
wherein the alarm mode is any one of: a receiver mode in which the receiver (30) outputs the alarm to the user; an autonomous mode in which the body-attachable device (10) outputs the alarm to the user; and a simultaneous mode in which the receiver (30) and the body-attachable device (10) output alarms to the user simultaneously.

2. The body-attachable unit (10) as claimed in claim 1, **characterized in that**, the alarm unit (10) is configured:
to determine whether the body-attachable device (10) and the receiver (30) are in communication with each other on the basis of whether a data request message is received from the receiver (30) in response to a pop-up message periodically transmitted via the communication unit (150).

3. The body-attachable unit (10) as claimed in claim 1 or 2, **characterized in that**, the alarm unit(130) further comprises:
an output unit (139), and
the alarm unit (130) is configured to generate a different alarm signal in accordance with the importance of the alarm and to output same through the output unit (139).

4. The body-attachable unit (10) as claimed in any one of the preceding claims,
**characterized in that**, in the case of a danger alarm, the device is configured such that the alarm condition can be set by input from the user.

5. The body-attachable unit (10) as claimed in any one of the preceding claims,
**characterized in that**, in the case of a danger alarm, the device is configured to set the alarm condition from previously measured biometric data.

6. The body-attachable unit (10) as claimed in any one of the preceding claims, **characterized in that**,
the alarm unit (130) is configured to, on the basis of the state of communication establishment with the receiver (30), perform control to output the alarm in the receiver mode when communication is established with the receiver (30), and perform control to output the alarm in the autonomous mode when the communication with the receiver (30) is interrupted.

7. The body-attachable unit (10) as claimed in claim 6, **characterized in that**, the alarm unit (130) is configured to:
perform control to output alarms in the simultaneous mode when communication is established with the receiver (30) and the importance of the alarm is determined to be high.

## Patentansprüche

1. Am Körper anbringbare Vorrichtung (10), die dazu konfiguriert ist, am Körper eines Benutzers angebracht zu werden, umfassend:
eine Sensoreinheit (110), die dazu konfiguriert ist, teilweise in den Körper des Benutzers einführbar zu sein, um biometrische Daten des Benutzers zu messen;
eine Kommunikationseinheit, die dazu konfiguriert ist, eine Kommunikation mit einem Empfänger (30) herzustellen und die gemessenen biometrischen Daten an den Empfänger (30) zu übertragen; und
eine Alarmeinheit (130), die dazu konfiguriert ist:
einen Zustand des Kommunikationsaufbaus zwischen der am Körper anbringbaren Vorrichtung (10) und dem Empfänger (30) zu bestimmen;
zu bestimmen, ob eine eingestellte Alarmbedingung auf der Grundlage eines Nutzungszustands der am Körper anbringbaren Vorrichtung oder der gemessenen biometrischen Daten erfüllt ist,
eine Wichtigkeit eines Alarms entsprechend dem Nutzungszustand der am Körper anbringbaren Vorrichtung (10) oder den gemessenen biometrischen Daten, welche die Alarmbedingung erfüllen, zu bestimmen,
wobei die Alarmbedingung eine beliebige der folgenden ist: eine Kalibrierungsalarmbedingung zum Anfordern der Eingabe eines Referenz-Blutzuckerwerts; eine Austauschalarmbedingung zum Anfordern des Austauschs der am Körper anbringbaren Vorrichtung (10); oder eine Gefahrenalarmbedingung zur Benachrichtigung über einen gefährlichen Zustand auf der Grundlage der biometrischen Daten; und
**dadurch gekennzeichnet, dass** die Alarmeinheit ferner dazu konfiguriert ist:
einen Alarmmodus auf der Grundlage des bestimmten Zustands des Kommunikationsaufbaus und der bestimmten Wichtigkeit des Alarms auszuwählen und, wenn die eingestellte Alarmbedingung erfüllt ist, dem Benutzer einen Alarm in dem ausgewählten Alarmmodus bereitzustellen,
wobei der Alarmmodus eine beliebige der folgenden Betriebsarten ist: ein Empfängermodus, in dem der Empfänger (30) den Alarm an den Benutzer ausgibt; ein autonomer Modus, in dem die am Körper anbringbare Vorrichtung (10) den Alarm an den Benutzer ausgibt; und ein Simultanmodus, in dem der Empfänger (30) und die am Körper anbringbare Vorrichtung (10) gleichzeitig Alarme an den Benutzer ausgeben.

2. Am Körper anbringbare Einheit (10) nach Anspruch 1, **dadurch gekennzeichnet, dass** die Alarmeinheit (10) dazu konfiguriert ist:
zu bestimmen, ob die am Körper anbringbare Vorrichtung (10) und der Empfänger (30) miteinander in Kommunikation stehen, auf der Grundlage dessen, ob von dem Empfänger (30) als Antwort auf eine über die Kommunikationseinheit (150) periodisch übertragene Pop-up-Nachricht eine Datenanforderungsnachricht empfangen wird.

3. Am Körper anbringbare Einheit (10) nach Anspruch 1 oder 2, **dadurch gekennzeichnet, dass** die Alarmeinheit (130) ferner umfasst:
eine Ausgabeeinheit (139), und
die Alarmeinheit (130) dazu konfiguriert ist, entsprechend der Wichtigkeit des Alarms ein unterschiedliches Alarmsignal zu erzeugen und dieses über die Ausgabeeinheit (139) auszugeben.

4. Am Körper anbringbare Einheit (10) nach einem der vorhergehenden Ansprüche,
**dadurch gekennzeichnet, dass** im Fall eines Gefahrenalarms die Vorrichtung so konfiguriert ist, dass die Alarmbedingung durch eine Eingabe des Benutzers eingestellt werden kann.

5. Am Körper anbringbare Einheit (10) nach einem der vorhergehenden Ansprüche,
**dadurch gekennzeichnet, dass** im Fall eines Gefahrenalarms die Vorrichtung dazu konfiguriert ist, die Alarmbedingung anhand zuvor gemessener biometrischer Daten einzustellen.

6. Am Körper anbringbare Einheit (10) nach einem der vorhergehenden Ansprüche,
**dadurch gekennzeichnet, dass**
die Alarmeinheit (130) dazu konfiguriert ist, auf der Grundlage des Zustands des Kommunikationsaufbaus mit dem Empfänger (30) eine Steuerung derart durchzuführen, dass der Alarm im Empfängermodus ausgegeben wird, wenn die Kommunikation mit dem Empfänger (30) hergestellt ist, und eine Steuerung derart durchzuführen, dass der Alarm im autonomen Modus ausgegeben wird, wenn die Kommunikation mit dem Empfänger (30) unterbrochen ist.

7. Am Körper anbringbare Einheit (10) nach Anspruch 6, **dadurch gekennzeichnet, dass** die Alarmeinheit (130) dazu konfiguriert ist:
eine Steuerung derart durchzuführen, dass Alarme im Simultanmodus ausgegeben werden, wenn die Kommunikation mit dem Empfänger (30) hergestellt ist und bestimmt wird, dass die Wichtigkeit des Alarms hoch ist.

## Revendications

1. Dispositif (10) pouvant être fixé sur le corps, prévu pour être fixé sur le corps d'un utilisateur, comprenant :
une unité de capteur (110) prévue pour être insérable en partie dans le corps de l'utilisateur afin de mesurer les données biométriques de l'utilisateur ;
une unité de communication prévue pour établir une communication avec un récepteur (30) et pour transmettre les données biométriques mesurées audit récepteur (30) ; et
une unité d'alarme (130) prévue pour :
déterminer un état d'établissement de communication entre le dispositif pouvant être fixé sur le corps (10) et le récepteur (30) ;
déterminer si une condition d'alarme définie est satisfaite sur la base d'une condition de service du dispositif pouvant être fixé sur le corps ou des données biométriques mesurées,
déterminer l'importance d'une alarme en fonction de la condition de service du dispositif pouvant être fixé sur le corps (10) ou des données biométriques mesurées qui satisfont à la condition d'alarme,
la condition d'alarme étant l'une quelconque des suivantes : une condition d'alarme d'étalonnage pour demander l'entrée d'une valeur de glycémie de référence ; une condition d'alarme de remplacement pour demander le remplacement du dispositif pouvant être fixé sur le corps (10) ; ou une condition d'alarme de danger pour la notification d'un état dangereux sur la base des données biométriques ;
**caractérisé en ce que** l'unité d'alarme est en outre prévue pour :
décider d'un mode d'alarme sur la base de l'état d'établissement de communication déterminé et de l'importance d'alarme déterminée, et émettre, si la condition d'alarme définie est satisfaite, une alarme vers l'utilisateur dans le mode d'alarme décidé,
le mode d'alarme étant l'un quelconque des modes suivants : un mode de récepteur dans lequel le récepteur (30) émet l'alarme vers l'utilisateur ; un mode autonome dans lequel le dispositif pouvant être fixé sur le corps (10) émet l'alarme vers l'utilisateur ; et un mode simultané dans lequel le récepteur (30) et le dispositif pouvant être fixé sur le corps (10) émettent simultanément des alarmes vers l'utilisateur.

2. Unité (10) pouvant être fixée sur le corps selon la revendication 1, **caractérisée en ce que** l'unité d'alarme (10) est prévue:
pour déterminer si le dispositif pouvant être fixé sur le corps (10) et le récepteur (30) sont en communication entre eux sur la base de la réception d'un message de demande de données provenant du récepteur (30) en réponse à un message contextuel transmis périodiquement par l'unité de communication (150).

3. Unité (10) pouvant être fixée sur le corps selon la revendication 1 ou la revendication 2, **caractérisée en ce que** l'unité d'alarme (130) comprend en outre :
une unité de sortie (139), et
**en ce que** l'unité d'alarme (130) est prévue pour générer un signal d'alarme différent en fonction de l'importance de l'alarme et pour émettre celui-ci via l'unité de sortie (139).

4. Unité (10) pouvant être fixée sur le corps selon l'une des revendications précédentes, **caractérisée en ce que**, dans le cas d'une alarme de danger, le dispositif est prévu de sorte que la condition d'alarme peut être définie par une entrée de l'utilisateur.

5. Unité (10) pouvant être fixée sur le corps selon l'une des revendications précédentes, **caractérisée en ce que**, dans le cas d'une alarme de danger, le dispositif est prévu pour définir la condition d'alarme à partir de données biométriques préalablement mesurées.

6. Unité (10) pouvant être fixée sur le corps selon l'une des revendications précédentes,
**caractérisée en ce que**
l'unité d'alarme (130) est prévue pour, sur la base de l'état d'établissement de communication avec le récepteur (30), exécuter une commande pour émettre l'alarme dans le mode de récepteur lorsque la communication est établie avec le récepteur (30), et exécuter une commande pour émettre l'alarme en mode autonome lorsque la communication avec le récepteur (30) est interrompue.

7. Unité (10) pouvant être fixée sur le corps selon la revendication 6, **caractérisée en ce que** l'unité d'alarme (130) est prévue pour :
exécuter une commande pour émettre des alarmes dans le mode simultané lorsque la communication est établie avec le récepteur (30) et que l'importance de l'alarme est déterminée comme élevée.
